## Europäisches Patentamt

(19) **European Patent Office**

**Office européen des brevets**

(11) Numéro de publication: **0 159 205**
**B1**

(12) **FASCICULE DE BREVET EUROPÉEN**

(45) Date de publication du fascicule du brevet:
**12.07.89**

(21) Numéro de dépôt: **85400172.4**

(22) Date de dépôt: **01.02.85**

(51) Int. Cl.⁴: **A 61 K 6/06**

(54) **Fritte pour couche céramique intermédiaire d'une reconstitution céramo-métallique dentaire.**

(30) Priorité: **15.02.84 FR 8402296**

(43) Date de publication de la demande:
**23.10.85 Bulletin 85/43**

(45) Mention de la délivrance du brevet:
**12.07.89 Bulletin 89/28**

(84) Etats contractants désignés:
**AT BE CH DE FR GB IT LI LU NL SE**

(56) Documents cité:
**DE-A-1 441 336**
**US-A-4 215 033**

**CHEMICAL ABSTRACTS, vol. 93, no. 10, 8 septembre 1980, page 393, no. 101507w, Columbus, Ohio, US; & JP - A - 80 03 301 (WADA SEIMITSU-SHIKEN CO., LTD.) 11-01-1980**
**DERWENT JAPANESE PATENTS REPORT, vol. U, no. 40, 6 novembre 1973, page 3, no. 59959u, Derwent Publications Ltd., Londres, GB; & JP - A - 73 31 558 (WADA SEIMITSU SHI-KEN CO., LTD.) 29-09-1973**

(73) Titulaire: **SUISSOR S.A., 9, rue Franklin, F-49024 Angers (FR)**

(72) Inventeur: **Heurtaux, Michel, 48a, rue Auguste Vacher, F-41200 Romorantin (FR)**

(74) Mandataire: **Ahner, Francis, CABINET REGIMBEAU 26, avenue Kléber, F-75116 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

## Description

La présente invention concerne le domaine technique des reconstitutions céramo-métalliques dentaires. Elle concerne plus particulièrement la composition du verre céramique utilisé pour réaliser différentes couches intermédiaires de reconstitutions dentaires, telles que des couronnes, incrustations, bridges, etc.

Ce type de reconstitution dentaire est habituellement obtenu à partir d'une succession de couches de céramique venant coiffer une chape métallique par exemple déposée sur le moignon d'une dent réduite après façonnage à la fraise.

Cette succession de couches céramiques se compose d'une couche basale d'opacification, de deux couches intermédiaires connues sous la dénomination de "dentine" et "incisal", et d'une couche superficielle, de préférence transparente, destinée à rappeler l'éclat de l'émail de la dent naturelle.

L'objet de la présente invention vise plus particulièrement des frittes de verre nécessaires à la réalisation d'une couche céramique intermédiaire à savoir, la dentine et/ou l'incisal.

Les nouvelles couches de céramique intermédiaires ont été mises au point dans le cadre de la présente invention pour permettre d'atteindre un certain nombre de caractéristiques recherchées, sans succès jusqu'à ce jour, par les utilisateurs de ce type de céramiques dentaires.

On a tout d'abord cherché à atteindre pour ces couches de verre céramique une température de cuisson plus basse, à savoir voisine d'environ 800°C. En effet, cet abaissement du point de fusion se traduit par une diminution de la dureté du verre céramique qui, dans l'état actuel de la technique, conduit en général à de très graves phénomènes d'abrasion des dents naturelles antagonistes.

Conformément à la présente invention, les couches céramiques intermédiaires sont destinées à recouvrir la couche céramique basale d'opacification de la chape métallique d'une reconstitution céramo-métallique dentaire; elles sont caractérisées en ce qu'elles sont obtenues à partir d'un mélange contenant au moins deux frittes de verre séparées, à savoir:

- environ 80 % en poids d'une première fritte de verre fusible et ne dévitrifiant pas, et
- environ 20 % en poids d'une seconde fritte de verre plus réfractaire et dévitrifiant partiellement en leucite,

ledit mélange ayant une granulométrie maximum inférieure à 65 μm et répondant à la composition globale suivante:

| | |
|---|---|
| $SiO_2$ | 59 à 59,5 % en poids |
| $Al_2O_3$ | 15 à 16 % en poids |
| CaO | 0,5 à 1,5 % en poids |
| MgO | 0 à 0,2 % en poids |
| $K_2O$ | 11 à 13 % en poids |
| $Na_2O$ | 7 à 8 % en poids |
| $B_2O_3$ | 2,5 à 3,5 % en poids |
| BaO | 0 à 3 % en poids |
| $CaF_2$ | 0,5 à 3 % en poids |
| $TiO_2$ | 0,2 à 0,5 % en poids |
| $CeO_2$ | environ 0,2 % en poids, |

ledit mélange entrant dans la composition d'une pâte prête à l'emploi contenant une quantité appropriée d'un agent plastifiant, apte à être conditionnée dans un tube ou un distributeur de pâte de type aérosol.

D'autres caractéristiques et avantages de la présente invention apparaîtront à la lecture de la description détaillée faite ci-après notamment en s'appuyant sur un exemple illustratif de réalisation pratique.

La présente invention a également eu pour objet la mise au point d'une couche céramique transparente présentant des coefficients de dilatation stables et adaptés à l'ensemble des alliages précieux ou non précieux.

Selon une caractéristique essentielle de la présente invention, la fritte de verre utilisée résulte d'un mélange de deux verres séparés, l'une fusible et ne dévitrifiant pas, l'autre légèrement plus réfractaire et dévitrifiant partiellement en leucite, minéral à forte dilatation thermique.

La première fritte de verre fusible et ne dévitrifiant pas est utilisée telle quelle. En revanche, la seconde fritte de verre est dévitrifiée par exemple pendant environ 12 heures à une température de l'ordre de 900°C. Au cours de ce traitement thermique de dévitrification, il apparaît de façon classique un phénomène de cristallisation in situ.

On observera en outre qu'au cours des cuissons successives, le verre fusible, à la limite de saturation en leucite ne dissout pas la leucite du deuxième verre et maintient donc son taux constant. Il en résulte ainsi une dilatation stable.

Bien entendu, la diminution de la dureté des couches céramiques intermédiaires selon la présente invention est également obtenue par augmentation du pourcentage de fondants, en privilégiant $Na_2O$. Les couches céramiques selon la présente invention sont en effet constituées à partir d'un mélange de deux frittes de verre contenant globalement d'environ 20 à environ 30 % en poids de fondants, parmi lesquels la soude $Na_2O$ est présente à raison d'environ 7 à environ 8 % en poids.

Les couches céramiques selon l'invention ont été également adaptées de façon à pouvoir éviter tout phénomène de coloration parasite provenant des supports métalliques. Ces supports métalliques sont habituellement réalisés à partir d'alliage de différents métaux parmi lesquels l'argent est en particulier responsable d'une telle coloration parasite. En effet, l'ion $Ag^{++}$ migre assez facilement dans la céramique et provoque une coloration jaune-verdâtre lorsqu'il se trouve réduit à l'état métallique. C'est précisément pour éviter ce type de phénomène, que les mélanges de frittes de verre selon l'invention sont additionnés d'environ 0,2 % en poids d'oxyde de cérium $CeO_2$ qui est un agent oxydant énergique maintenant l'argent à l'état d'oxyde non colorant. Cet oxyde de cérium est ajouté après l'opération de broyage conduisant à la fritte de verre. Les frittes de verre sont obtenues de façon classique par fusion et homogénéisation d'un mélange pulvérulent des constituants de base nécessaires à la réalisation des compositions précitées.

Pour diminuer la rétraction au cours de la cuisson, la répartition granulométrique du mélange des deux frittes de verre a été ajustée à la suite de l'observation suivante. La porosité d'un ensemble de gros grains (40 à 65 μm) est de l'ordre de 40 %. En introduisant des grains de dimensions moyennes (12 à 40 μm) à raison d'environ 50 % en poids par rapport aux gros grains, la porosité chute à environ 20 %. Enfin, si on introduit des grains de dimensions beaucoup plus fins (inférieurs à 5 μm), la porosité devient encore plus faible. Il en résulte donc un retrait de cuisson moins important. Un tel type de répartition granulométrique permet également d'obtenir une meilleure plasticité de la pâte au moment de son utilisation.

Pour la réalisation d'une fritte de verre selon l'invention destinée à la réalisation d'une couche superficielle transparente, il est ainsi souhaitable de faire appel à un profil granulométrique du type suivant:

40 à 65 μm    environ 4/7 des grains
12 à 40 μm    environ 2/7 des grains
< 12 μm       environ 1/7 des grains.

En revanche, pour la réalisation d'une fritte de verre destinée à constituer l'incisal, qui se caractérise par rapport à la dentine par une translucidité plus importante, il sera préférable de diminuer la proportion de grains les plus fins. Dans la pratique, un profil granulométrique du type suivant s'est avéré tout à fait satisfaisant:

40 à 65 μm    environ 8,5/14 des grains
12 à 40 μm    environ 4,5/14 des grains
< 12 μm       environ 1/14 des grains.

La présente invention concerne également les frittes de verre nécessaires à la réalisation des couches céramiques superficielles transparentes, qui sont présentées sous la forme d'une pâte prête à l'emploi destinée à faciliter le travail de l'utilisateur tout en permettant de réaliser une économie de produit actif. De telles frittes se présentent sous la forme d'une pâte contenant une quantité appropriée d'un agent plastifiant de manière à permettre leur conditionnement dans un tube ou un distributeur de pâte de type aérosol.

On indiquera ci-après un exemple type de formulation de frittes de verre selon l'invention conditionnées dans un distributeur aérosol:

100 parties en poids de produits actifs,
30 à 40 parties en poids d'agent plastifiant,
100 parties en poids d'un agent propulseur, tel qu'un Freon®.

Conformément à un mode de réalisation particulier de ce type de frittes de verre, l'agent plastifiant est de préférence choisi parmi l'éther diéthylique du diéthylèneglycol et l'éther méthylique du propylèneglycol.

Selon une variante avantageuse de la présente invention, la dentine de base est décomposée en deux dentines distinctes, à savoir une dentine opaque et une dentine translucide. La dentine opaque, moins saturée en teinte que la dentine transparente, contient un pourcentage déterminé d'opacifiant, par exemple constitué par une fritte de verre opacifiée ou encore de la zircone. La dentine transparente est en revanche très colorée et très faiblement opacifiée. Ainsi par juxtaposition et/ou par mélange de ces deux types de dentine, l'utilisateur pourra mieux restituer les zones de contrastes multiples présentes dans une dent naturelle. Bien entendu, ces différentes compositions de dentine pourront être conditionnées en mélange avec des colorants organiques qui permettent de visualiser la construction de la dent et qui s'éliminent au cours de la cuisson.

C'est ainsi que les pâtes de frittes de verre selon l'invention peuvent être additionnées de différents colorants, afin de pouvoir mettre à la disposition du prothésiste une gamme complète de coloration de couches céramiques s'adaptant aux teintes des dents naturelles de différents patients.

**Revendications**

1. Frittes de verre nécessaires à la réalisation d'une couche céramique intermédiaire constituant la dentine et/ou l'incisal, destinée à recouvrir la couche céramique basale d'opacification de la chape métallique d'une reconstitution céramo-métallique dentaire, caractérisées en ce qu'elles sont obtenues à partir d'un mélange contenant au moins deux frittes de verre séparées, à savoir:

. environ 80 en poids d'une première fritte de verre fusible et ne dévitrifiant pas, et

. environ 20 en poids d'une seconde fritte de verre plus réfractaire et dévitrifiant partiellement en leucite, ledit mélange ayant une granulométrie maximum inférieure à 65 μm et répondant à la composition globale suivante:

| | |
|---|---|
| $SiO_2$ | 59 à 59,5 % en poids |
| $Al_2O_3$ | 15 à 16 % en poids |
| CaO | 0,5 à 1,5 % en poids |
| MgO | 0 à 0,2 % en poids |
| $K_2O$ | 11 à 13 % en poids |
| $Na_2O$ | 7 à 8 % en poids |
| $B_2O_3$ | 2,5 à 3,5 % en poids |
| BaO | 0 à 3 % en poids |
| $CaF_2$ | 0,5 à 3 % en poids |

TiO$_2$    0,2 à 0,5 % en poids
CeO$_2$    environ 0,2 % en poids,

ledit mélange entrant dans la composition d'une pâte prête à l'emploi contenant une quantité appropriée d'un agent plastifiant, apte à être conditionnée dans un tube ou un distributeur de pâte de type aérosol.

2. Fritte de verre nécessaire à la réalisation de la dentine selon la revendication 1, caractérisée en ce que ledit mélange des deux frittes de verre présente un profil granulométrique du type suivant:

40 à 65 μm    environ 4/7 des grains
12 à 40 μm    environ 2/7 des grains
< 12 μm       environ 1/7 des grains.

3. Fritte de verre nécessaire à la réalisation de l'incisal selon la revendication 1, caractérisée en ce que ledit mélange des deux frittes présente un profil granulométrique du type suivant:

40 à 65 μm    environ 8,5/14 des grains
12 à 40 μm    environ 4,5/14 des grains
< 12 μm       environ 1/14 des grains.

4. Frittes de verre selon les revendications 1 à 3, caractérisées en ce que l'agent plastifiant est choisi parmi l'éther diéthylique du diéthylèneglycol et l'éther méthylique du propylèneglycol.

**Patentansprüche**

1. Glasfritten, die zur Ausbildung einer keramischen Zwischenschicht benötigt werden, die das Zahnbein und/oder die Einlage zum Überdecken der keramischen Grundtrübungsschicht der Metallkrone eines keramisch-metallischen Zahnwiederaufbaus bildet, dadurch <u>gekennzeichnet</u>, daß sie ausgehend von einem Gemisch erhalten wird, das wenigstens zwei getrennte Glasfritten, nämlich etwa 80 Gewichtsprozent einer ersten schmelzbaren, nicht entglasbaren Glasfritte und etwa 20 Gewichtsprozent einer zweiten, hochschmelzenden und mit Leucit partiell entglasbaren Glasfritte enthält, wobei dieses Gemisch eine maximale Korngröße von weniger als 65 μm und die folgende Gesamtzusammensetzung hat:

SiO$_2$    59 bis 59,5 Gewichtsprozent,
Al$_2$O$_3$  15 bis 16 Gewichtsprozent
CaO     0,5 bis 1,5 Gewichtsprozent
MgO     0 bis 0,2 Gewichtsprozent
K$_2$O    11 bis 13 Gewichtsprozent
Na$_2$O   7 bis 8 Gewichtsprozent
B$_2$O$_3$   2,5 bis 3,5 Gewichtsprozent
BaO     0 bis 3 Gewichtsprozent
CaF$_2$   0,5 bis 3 Gewichtsprozent
TiO$_2$    0,2 bis 0,5 Gewichtsprozent
CeO$_2$    etwa 0,2 Gewichtsprozent,

und wobei dieses Gemisch die Form einer verwendungsfertigen Paste einnimmt, die eine angemessene Menge eines Plastifikationsmittels enthält und somit in einer Tube oder einem Pastenspender vom Aerosoltyp aufgenommen werden kann.

2. Glasfritte, die zur Ausbildung des Zahnbeines benötigt wird, nach Anspruch 1, dadurch <u>gekennzeichnet</u>, daß das besagte Gemisch der beiden Glasfritten ein Korngrößenprofil folgenden Typs hat:

40 bis 65 μm    etwa 4/7 der Körner
12 bis 40 μm    etwa 2/7 der Körner
< 12 μm         etwa 1/7 der Körner.

3. Glasfritte, die zur Ausbildung der Einlage benötigt wird, nach Anspruch 1, dadurch <u>gekennzeichnet</u>, daß das besagte Gemisch der beiden Glasfritten ein Korngrößenprofil folgenden Typs hat:

40 bis 65 μm    etwa 8,5/14 der Körner
12 bis 40 μm    etwa 4,5/14 der Körner
< 12 μm         etwa 1/14 der Körner.

4. Glasfritten nach Anspruch 1 bis 3, dadurch <u>gekennzeichnet</u>, daß das Plastifikationsmittel aus Diethyldiethylenglykol oder Methylpropylenglykol gewählt ist.

**Claims**

1. Glass frits required for producing an intermediary ceramic layer forming the dentine and/or the incisal, intended to cover the basal ceramic layer for opacifying the metal coping of a dental ceramo-metallic reconstruction, characterized in that they are obtained from a mixture containing at least two separate glass frits, namely:

- approximately 80 % by weight of a first glass frit which is fusible and does not devitrify, and

- approximately 20 % by weight of a second glass frit which is more refractory and devitrifies partially to leucite, the said mixture having a maximum particle size of less than 65 μm and corresponding to the following overall composition:

SiO$_2$    59 to 59.5 % by weight
Al$_2$O$_3$  15 to 16 % by weight
CaO     0.5 to 1.5 by weight
MgO     0 to 0.2 % by weight
K$_2$O    11 to 13 % by weight
Na$_2$O   7 to 8 % by weight
B$_2$O$_3$   2.5 to 3.5 % by weight
BaO     0 to 3 % by weight
CaF$_2$   0.5 to 3 % by weight
TiO$_2$    0.2 to 0.5 % by weight
CeO     approximately 0.2 % by weight,

the said mixture forming part of the composition of a ready-for-use paste containing an appropriate quantity of a plasticizing agent and capable of being packaged in a tub or a paste dispenser of the aerosol type.

2. Glass frit required for producing the dentine according to Claim 1, characterized in that the said mixture of the two glass frits has a particle size distribution of the following type:

40 to 65 μm   approximately 4/7 of the particles
12 to 40 μm   approximately 2/7 of the particles
< 12 μm      approximately 1/7 of the particles.

3. Glass frit required for producing the incisal according to Claim 1, characterized in that the said mixture of the two frits has a particle size distribution of the following type:

40 to 65 μm   approximately 8.5/14 of the particles
12 to 40 μm   approximately 4.5/14 of the particles
< 12 μm      approximately 1/14 of the particles.

4. Glass frits according to Claims 1 to 3, characterized in that the plasticizing agent is chosen from diethylene glycol diethyl ether and propylene glycol methyl ether.